# EUROPÄISCHE PATENTSCHRIFT

(11) **EP 2 523 707 B1**
(45) Veröffentlichungstag und Bekanntmachung des Hinweises auf die Patenterteilung: **19.11.2014**
(21) Anmeldenummer: 10805604.5
(22) Anmeldetag: 23.12.2010
(51) Int. Cl.: A61M 5/20, A61M 5/46

(54) **EINSTECHTIEFENEINSTELLUNG**
SETTING OF PIERCING DEPTH
RÉGLAGE DE PROFONDEUR DE PIQUAGE

(30) Priorität: 13.01.2010 DE 202010000846 U
(43) Veröffentlichungstag der Anmeldung: 21.11.2012
(73) Patentinhaber: Bayer Pharma AG, 13353 Berlin (DE)
(72) Erfinder: WEBER, Wilfried, 72296 Schopfloch (DE); RENZ, Andreas, 72172 Sulz am Neckar (DE); PETRY, Dariusz, 71034 Böblingen (DE)
(74) Vertreter: BIP Patents
(86) Internationale Anmeldenummer: PCT/EP2010/007929
(87) Internationale Veröffentlichungsnummer: WO 2011/085797

(56) Entgegenhaltungen:
- EP-A2- 0 238 378
- DE-U1-202009 001 836
- US-A1- 2001 047 151

## Beschreibung

Die Erfindung betrifft eine Injektionsvorrichtung für medizinische Anwendungen nach dem Oberbegriff des Anspruchs 1. Diese Injektionsvorrichtung weist ein Trägergehäuse auf, in dem ein Injektionsgerät, wie eine Spritze oder eine Karpule, mit wenigstens einem auspressbaren Injektionsflüssigkeitsbehälter einsetzbar ist. Zudem weist die Injektionsvorrichtung eine zur Aktivierung des Injektionsgerätes entlang einer Injektionsrichtung antreibbare Betätigungsvorrichtung auf, die einen Einstechschlitten, eine Aufnahme für das Injektionsgerät sowie einen relativ zum Einstechschlitten verlagerbaren Injektionsschlitten aufweist. Der Injektionsschlitten führt dabei einen Betätigungsstößel mit sich, mittels dem ein Stößel eines eingesetzten Injektionsgerätes beaufschlagt werden kann. Zumindest zur Durchführung eines Einstechhubes, bei dem eine Einstechnadel des eingesetzten Injektionsgerätes aus dem Trägergehäuse heraus gefahren werden kann, und eines Injektionshubes, bei dem eine Flüssigkeit aus dem Injektionsflüssigkeitsbehälter heraus gepresst werden kann, sind der Einstechschlitten und der Injektionsschlitten dabei von einer Kraftbeaufschlagungseinrichtung betätigbar. Ferner sind an der Injektionsvorrichtung Mittel zur Einstellung einer Einstechtiefe vorgesehen, bei deren Erreichen der Einstechhub beendet und der Injektionshub eingeleitet werden kann.

Aus der DE 20 2009 001 836 U1 ist eine elektromechanische Injektionsvorrichtung für medizinische Anwendungen bekannt. Diese weist einen Einstechtiefenanschlag auf, der den Bewegungspfad eines Einstechschlittens begrenzt und dadurch eine Einstechtiefe bestimmt. Der Einstechtiefenanschlag ist dabei über eine von außerhalb des Trägergehäuses betätigbare Stellschraube translatorisch verlagerbar.

Der Vorteil derartiger Injektionsvorrichtungen besteht darin, dass die Einstechtiefe besonders einfach und somit beispielsweise auch durch den Patienten selbst eingestellt werden kann. Allerdings ist für die Einstellbarkeit der Einstechtiefe eine recht komplexe Mechanik erforderlich, da der Einstechtiefenanschlag in seiner jeweiligen Stellung auf die übrigen Mittel zur Steuerung des Einstech- und Injektionshubes abgestimmt sein muss.

Die Aufgabe der Erfindung besteht darin eine Injektionsvorrichtung bereit zu stellen, die bei vereinfachtem Aufbau eine sichere und einfache Einstellung der Einstechtiefe ermöglicht.

Diese Aufgabe wird durch eine Injektionsvorrichtung mit den Merkmalen des Anspruchs 1 gelöst. Dabei ist die Aufnahme zur Einstellung der gewünschten Einstechtiefe in Injektionsrichtung verstellbar am Einstechschlitten gelagert. Auf diese Weise wird die Einstechtiefe, über die eine Injektionsnadel eines in die Injektionsvorrichtung eingesetzten Injektionsgerätes bei einer vorgesehenen Anwendung aus dem Trägergehäuse heraus verlagerbar ist, allein durch die eingestellte Position der Aufnahme gegenüber dem Einstechschlitten bestimmt sowie durch einen gleich bleibenden Endanschlag des Einstechschlittens gegenüber dem Trägergehäuse. Die sonstigen Mittel zur Steuerung des Einstech- oder Injektionshubes können auf diese Weise zumindest weitestgehend unabhängig von der eingestellten Einstechtiefe ausgelegt werden, was wiederum einen einfacheren Aufbau der Injektionsvorrichtung ermöglicht.

Vorteilhafterweise sind Festlegemittel vorgesehen, mittels denen die Aufnahme in einer eingestellten beziehungsweise ausgewählten Position gegenüber dem Einstechschlitten festlegbar ist. Auf diese Weise kann die gewünschte Einstechtiefe dauerhaft eingestellt werden.

Zudem ist es günstig, wenn die Festlegemittel wenigstens ein Eingriffselement aufweisen, das in einer von mehreren vorbestimmten Eingriffspositionen mit entsprechenden Eingriffsgegenelementen in Eingriff bringbar ist, wodurch eine besonders stabile Festlegung der Aufnahme gegenüber dem Einstechschlitten und damit der eingestellten Einstechtiefe gewährleistet ist.

In einer besonders vorteilhaften Ausführungsform der Injektionsvorrichtung ist der Eingriff über einen Verriegelungsschieber herstellbar, der zwischen einer Verriegelungsstellung, in der Verriegelungsmittel des Verriegelungsschiebers mit Verriegelungsgegenmitteln des Einstechschlittens in Eingriff stehen, und einer Freigabestellung, in der die Verriegelungsmittel und Verriegelungsgegenmittel außer Eingriff stehen, verschiebbar ist. Hierdurch ist die lösbare Festlegung der Aufnahme am Einstechschlitten mit besonders einfachen Mitteln möglich.

Dabei ist es günstig, wenn der Verriegelungsschieber in die Verriegelungsstellung vorgespannt ist, um die Aufnahme so lange gegenüber dem Einstechschlitten festzulegen bis eine andere Einstechtiefe eingestellt wird.

In einer hierzu alternativen Ausführungsform der Injektionsvorrichtung ist der Verriegelungsschieber dagegen in die Freigabestellung vorgespannt. Auf diese Weise kann der jeweilige Anwender vor jeder Benutzung dazu angehalten werden, bewusst eine bestimmte Einstechtiefe einzustellen, die der jeweils vorgesehenen Anwendung entspricht.

Hierbei ist es besonders vorteilhaft, wenn der Verriegelungsschieber über eine Handhabe verlagerbar ist, die in der Freigabestellung in einen Aufnahmeraum der Aufnahme ragt, das heißt, dass die Aufnahme durch die Handhabe blockierbar ist. Somit kann eine eingestellte Einstechtiefe nur dann verstellt werden kann, wenn in der Aufnahme kein Injektionsgerät festgelegt ist. Im Falle der Ausführungsform mit dem in die Freigabestellung vorgespannten Verriegelungsschieber erfolgt bei Einsetzen eines Injektionsgerätes in die Aufnahme zudem die Fixierung der Aufnahme am Einstechschlitten beziehungsweise der dadurch vorgegebenen Einstechtiefe.

Zudem ist es günstig, wenn die Handhabe durch einen ersten Arm eines zweiarmigen Schwenkgliedes gebildet ist, das um eine Schwenkachse herum verschwenkbar gelagert ist und an einem zweiten Arm einen exzentrischen Nocken zur Verlagerung des Verriegelungsschiebers aufweist, wodurch eine komfortable Betätigung der Handhabe entgegen der jeweiligen Vorspannung möglich ist. Vorteilhafterweise ist am Einstechschlitten zudem eine Anzeige zur Bestimmung der aktuell eingestellten Einstechtiefe vorgesehen, was das Einstellen einer von mehreren möglichen vorgegebenen Einstechtiefen weiter vereinfacht.

Dabei ist es günstig, wenn die Anzeige eine sich in Injektionsrichtung erstreckende Skala aufweist, an der entlang ein Zeigerelement der Aufnahme verschiebbar ist, wodurch eine aktuelle Position der Aufnahme gegenüber dem Einstechschlitten beziehungsweise die hierdurch vorgegebene Einstechtiefe besonders gut ablesbar ist.

In einer alternativen besonders vorteilhaften Ausführungsform der Injektionsvorrichtung sind die Aufnahme und der Einstechschlitten über ein Stellgetriebe zueinander verlagerbar, wobei das Stellgetriebe über ein von außerhalb des Trägergehäuses zugängliches Stellrad betätigt werden kann. Hierdurch ist die Position der Aufnahme gegenüber dem Einstechschlitten und die dadurch vorgegebene Einstechtiefe für den Benutzer in besonders komfortabler Weise einstellbar.

Dabei ist es günstig, wenn das Stellgetriebe eine Spindel aufweist, die über einen Zahnradabschnitt direkt oder indirekt mit dem Stellrad kämmt und über einen Gewindeabschnitt mit einer Mitnahmekontur der Aufnahme in Eingriff steht. Ferner ist die Spindel verdrehbar am Einstechschlitten gelagert. Eine derartige Spindel ermöglicht eine besonders einfache und kostengünstige Herstellung des Stellgetriebes.

Hierbei ist es besonders vorteilhaft, wenn der Zahnradabschnitt in kämmender Position mit dem Stellrad an demselben mittels Gleitbewegung verlagerbar ist. Auf diese Weise bildet das Stellrad hinsichtlich des Zahnradabschnittes eine Art Gleitlager, das es ermöglicht, dass die kämmende Stellung beider Elemente zueinander auch während eines Einstechhubes erhalten bleiben kann, was wiederum den Aufbau der Injektionsvorrichtung vereinfacht.

Zudem ist es vorteilhaft, wenn das Eingriffselement hierbei durch eine gehäuseseitige Rastnase gebildet ist, die mit einer von mehreren in das Stellrad eingelassenen Rastaufnahmen verrastbar ist, wodurch eine sichere Festlegung einer eingestellten Drehposition des Stellrades beziehungsweise der entsprechenden translatorischen Position der Aufnahme gegenüber dem Einstechschlitten und der entsprechend vorgegebenen Einstechtiefe gewährleistet werden kann.

In den Figuren ist eine beispielhafte Ausführungsform der Erfindung dargestellt. Es zeigen:
- Figur 1: eine perspektivische Ansicht einer erfindungsgemäßen Injektionsvorrichtung mit eingesetztem Injektionsgerät,
- Figur 2: einen Einstechschlitten der Injektionsvorrichtung nach Fig. 1 mit leerer Aufnahme,
- Figur 3a: eine Draufsicht auf den Einstechschlitten nach Fig. 2,
- Figur 3b: eine Seitenansicht des Einstechschlittens in Richtung IIIb aus Fig. 3a,
- Figur 4a: einen Schnitt in Ebene IV.a aus Fig. 3b,
- Figur 4b: einen Schnitt in Ebene IV.b aus Fig. 3a,
- Figur 5: eine perspektivische Darstellung des Einstechschlittens nach Fig. 2 in einer Freigabestellung,
- Figur 6a: eine Draufsicht auf den Einstechschlitten nach Fig. 5,
- Figur 6b: einen Schnitt in Ebene VI.b aus Fig. 6a,
- Figur 7: eine perspektivische Ansicht einer alternativen Ausführungsform der erfindungsgemäßen Injektionsvorrichtung mit eingesetztem Injektionsgerät,
- Figur 8: eine perspektivische freigeschnittene Ansicht eines Stellgetriebes der Injektionsvorrichtung nach Fig. 7,
- Figur 9a: eine Draufsicht auf einen Einstechschlitten der Injektionsvorrichtung nach Fig. 7 mit leerer Aufnahme,
- Figur 9b: einen Schnitt in Ebene IX.b aus Fig. 9a und
- Figur 10: eine perspektivische freigestellte Ansicht eines mit einer Spindel kämmenden Stellrades des Stellgetriebes nach Fig. 8.

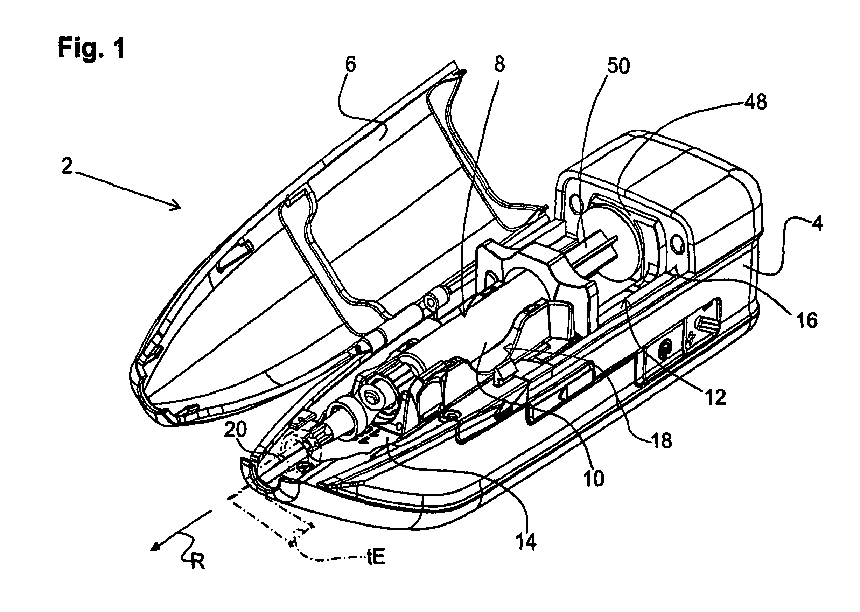

Fig. 1 zeigt eine erfindungsgemäße Injektionsvorrichtung 2 mit einem Trägergehäuse 4 bei geöffnetem Deckel 6. In die Injektionsvorrichtung 2 ist ein spritzenförmiges Injektionsgerät 8 mit einem Injektionsflüssigkeitsbehälter 10 eingesetzt. Dabei weist die Injektionsvorrichtung 2 zur Aufnahme und Verlagerung des Injektionsgerätes 8 entlang einer Injektionsrichtung R eine Betätigungsvorrichtung 12 auf, die einen Einstechschlitten 14 mit einer Aufnahme 18, in der das Injektionsgerät 8 einsetzbar ist, und einen Injektionsschlitten 16 aufweist, über den das Injektionsgerät 8 auspressbar ist und der gegenüber dem Einstechschlitten 14 verlagerbar ist.

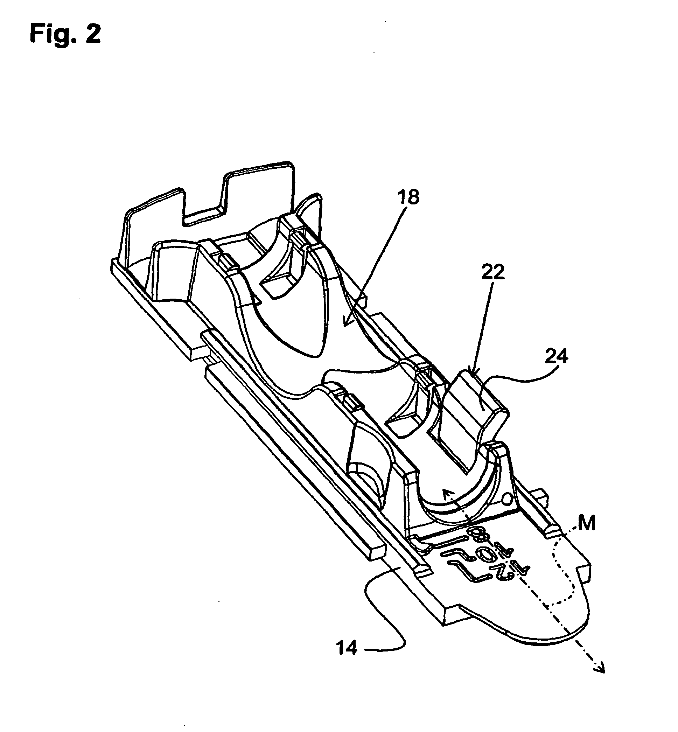

Um eine für eine vorgesehene Anwendung vorbestimmte Einstechtiefe tE einstellen zu können, über die eine Einstechnadel 20 des Injektionsgerätes 8, wie durch strichpunktierte Linien dargestellt, aus dem Trägergehäuse 4 heraus gefahren werden kann, ist die Aufnahme 18 gegenüber dem übrigen Einstechschlitten 14 parallel zur Injektionsrichtung R verschiebbar gelagert, wie durch Pfeil M in Fig. 2 dargestellt.

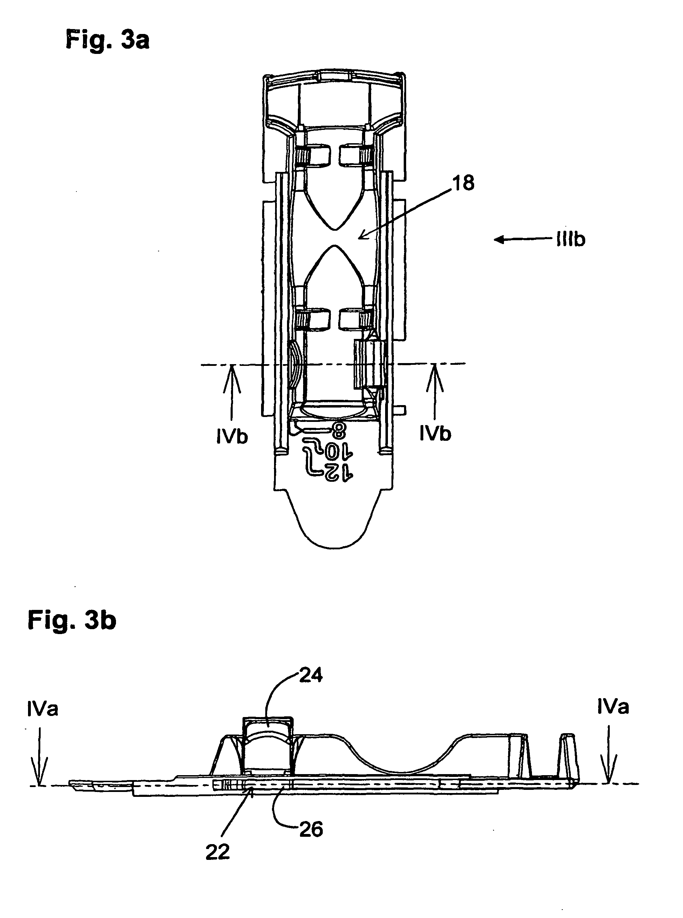

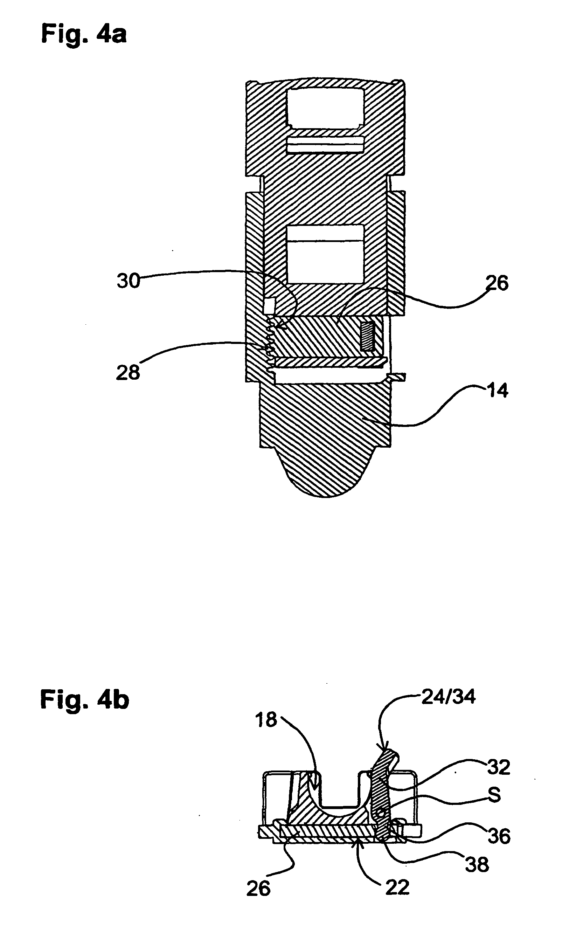

Wie aus Fig. 3a, 3b, 4a und 4b zu entnehmen ist, sind an der Aufnahme 18 Festlegemittel 22 vorgesehen, mittels denen diese in einer von mehreren Positionen am Einstechschlitten festlegbar ist. Die Festlegemittel 22 weisen dabei ein Eingriffselement in Form eines über eine Handhabe 24 verstellbaren Verriegelungsschiebers 26 auf, der an einem von der Handhabe 24 abgewandten Ende Verriegelungsmittel 28 in Form von Zähnen aufweist. Diese Verriegelungsmittel 28 sind mit Verriegelungsgegenmitteln 30 in Eingriff bringbar, die komplementär zu den Verriegelungsmitteln 28 ausgebildet sind, wie insbesondere aus Fig. 4a zu entnehmen ist. Auf diese Weise bilden die Verriegelungsgegenmittel 30, die als Zahnungsabschnitt ausgebildet sind, ein Eingriffsgegenelement, an dem der Verriegelungsschieber 26 in eine von mehreren möglichen Eingriffspositionen in Eingriff bringbar ist. Auf diese Weise nehmen der Einstechschlitten 14 und die Aufnahme 18 zueinander eine Verriegelungsstellung ein, in der die Aufnahme 18 am übrigen Einstechschlitten 14 festgelegt ist.

Wie insbesondere aus Fig. 4b zu entnehmen ist, ist die Handhabe 24 durch einen ersten Arm 32 eines zweiarmigen Schwenkgliedes 34 gebildet, das um eine Schwenkachse S herum verschwenkbar an der Aufnahme 18 gehalten ist. Ein zweiter Arm 36 des Schwenkgliedes 34 bildet einen exzentrischen Nocken 38 aus, der mit dem Verriegelungsschieber 26 in Eingriff steht.

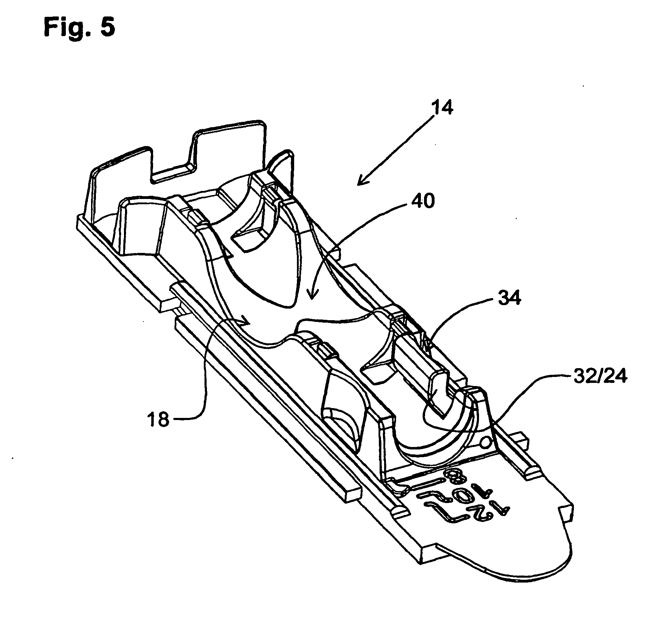

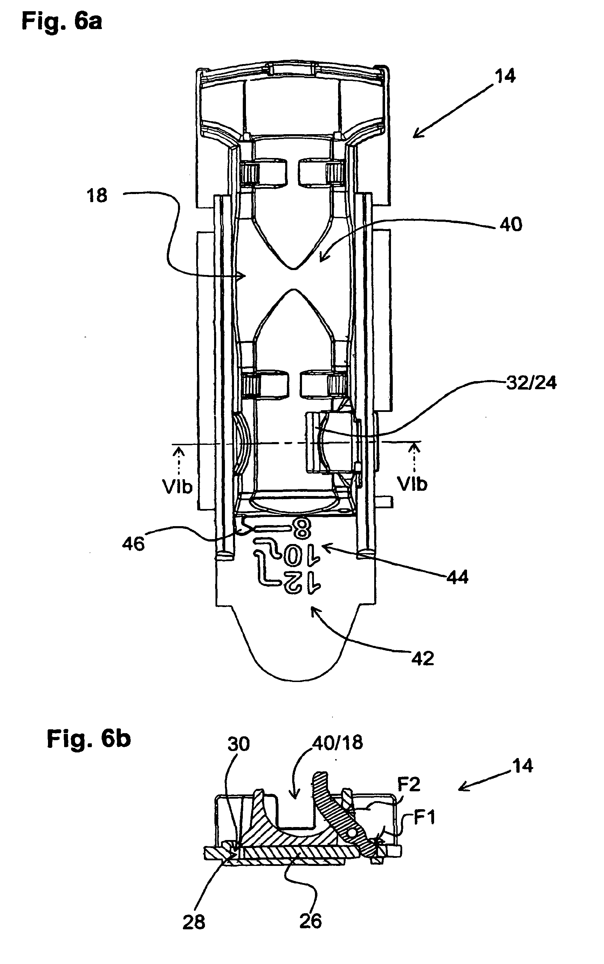

Durch Verschwenken des ersten Armes 32 um die Schwenkachse S herum in einen durch die Aufnahme 18 teilweise begrenzten Aufnahmeraum 40 hinein, ist der Einstechschlitten 14 in eine Freigabestellung verbringbar, wie sie in den Fig. 5, 6a und 6b dargestellt ist. Bei diesem Verschwenken des Schwenkgliedes 34 werden die Verriegelungsmittel 28 des Verriegelungsschiebers 26 außer Eingriff mit den Verriegeiungsgegenmitteln 30 gebracht (siehe Fig. 6b), so dass die Aufnahme 18 zur Einstellung einer neuen Einstechtiefe tE gegenüber dem übrigen Einstechschlitten 14 in Injektionsrichtung R verschiebbar ist.

Um hierbei für die Einstechtiefe tE einen genauen Wert einstellen zu können, ist an dem Einstechschlitten 14, wie insbesondere aus Fig. 6a zu entnehmen ist, eine Anzeige 42 vorgesehen, die eine Skala 44 aus mehreren einstellbaren Werten aufweist, entlang der ein Zeigerelement 46 verfahrbar ist, das mit der Aufnahme 18 fest verbunden ist.

Sobald die Aufnahme 18 derart gegenüber dem übrigen Einstechschlitten 14 verschoben ist, dass das Zeigerelement 46 auf einen gewünschten Wert der Skala 44 zeigt, wird die Handhabe 24 wieder aus dem Aufnahmeraum 40 heraus geschwenkt und dadurch die Verriegelungsmittel 28 des Verriegelungsschiebers 26 in der entsprechenden Eingriffsposition mit den Verriegelungsgegenmitteln 30 in Eingriff gebracht und folglich die Verriegelungsstellung zwischen dem Einstechschlitten 14 und der Aufnahme 18 wieder hergestellt. Durch Einsetzen des Injektionsgerätes 8 in die Aufnahme 18 kann die Handhabe 24 dann soweit blockiert werden, dass sie nicht mehr in den Aufnahmeraum 40 verschwenkbar ist. Auf diese Weise ist somit auch der Verriegelungsschieber 26 in der Eingriffstellung mit den Verriegelungsgegenmitteln 30 festgelegt. Um den Wert der eingestellten Injektionstiefe tE erneut verstellen zu können müsste somit also zunächst das Injektionsgerät 8 wieder aus der Aufnahme 18 entnommen werden.

Ferner kann an dem Schwenkglied 34, wie in Fig. 6b durch Pfeile F1 und F2 angedeutet, eine Federkraft vorgesehen sein, die dieses in eine Endstellung vorspannt.

Bei der Ausführungsform mit der Federkraft F1 wird das Schwenkglied 34 dabei in die Verriegelungsstellung gemäß Fig. 2 bis 4b vorgespannt, so dass eine eingestellte Einstechtiefe tE permanent fixiert bleibt, bis mittels Betätigung der Handhabe 24 und Verschieben der Aufnahme 18 gegenüber dem übrigen Einstechschlitten 14 eine neue Einstechtiefe tE eingestellt wird.

Alternativ hierzu ist das Schwenkglied 34 bei der Ausführungsform mit der Federkraft F2 in die Freigabestellung gemäß Fig. 5 bis 6b vorgespannt. Hierdurch wird ein Benutzer dazu angehalten, bei Einsetzen eines Injektionsgerätes 8 in die Aufnahme 18 eine bestimmte Einstechtiefe tE bewusst einzustellen.

Nach dem Einstellen der Einstechtiefe tE und dem Einsetzen des Injektionsgerätes 8 kann dann die Injektionsvorrichtung 2 bei geschlossenem Deckel 6 ausgelöst werden. Hierbei wird die Betätigungsvorrichtung 12 derart von einer nicht näher dargestellten beispielsweise rein mechanischen oder elektromechanischen Kraftbeaufschlagungseinrichtung der Betätigungsvorrichtung 12 derart beaufschlagt, dass während eines Einstechhubes der Einstechschlitten 14 und der Injektionsschlitten 16 gemeinsam in Injektionsrichtung R verlagert werden bis die Einstechnadel 20 über die Einstechtiefe tE hinweg aus dem Trägergehäuse 4 heraus ragt. Anschließend wird dann lediglich der Injektionsschlitten 16 in Injektionsrichtung R weiter bewegt, wobei ein Betätigungsstößel 48 des Injektionsschlittens 16 (gemäß Fig. 1) gegen einen Stößel 50 des Injektionsgerätes 8 drückt, über den dadurch der Injektionsflüssigkeitsbehälter 10 ausgepresst wird.

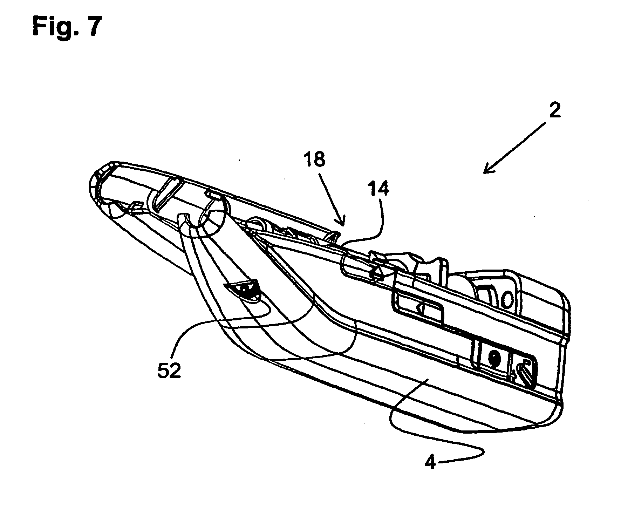

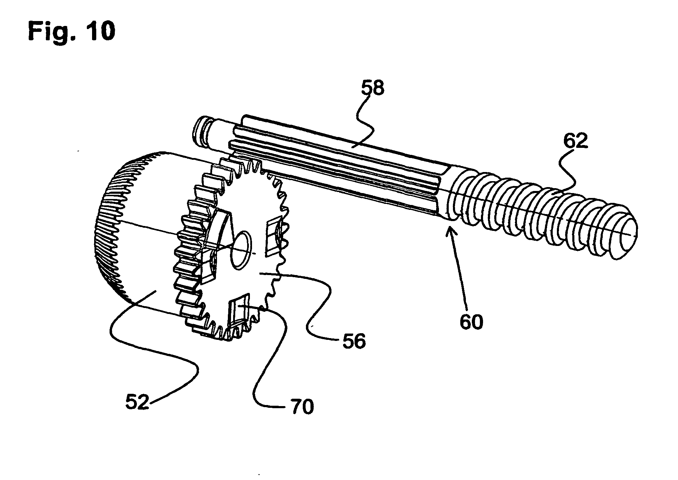

Fig. 7 bis 10 zeigen eine alternative Ausführungsform der Injektionsvorrichtung 2, bei der die Aufnahme 18 mittels eines von außerhalb des Trägergehäuses 4 betätigbaren Stellrades 52 gegenüber dem übrigen Einstechschlitten 14 verstellbar ist, um die gewünschte Einstechtiefe tE einzustellen. Im Übrigen stimmt die Injektionsvorrichtung 2 mit der hinsichtlich Fig. 1 bis 6b beschriebenen Ausführungsform überein.

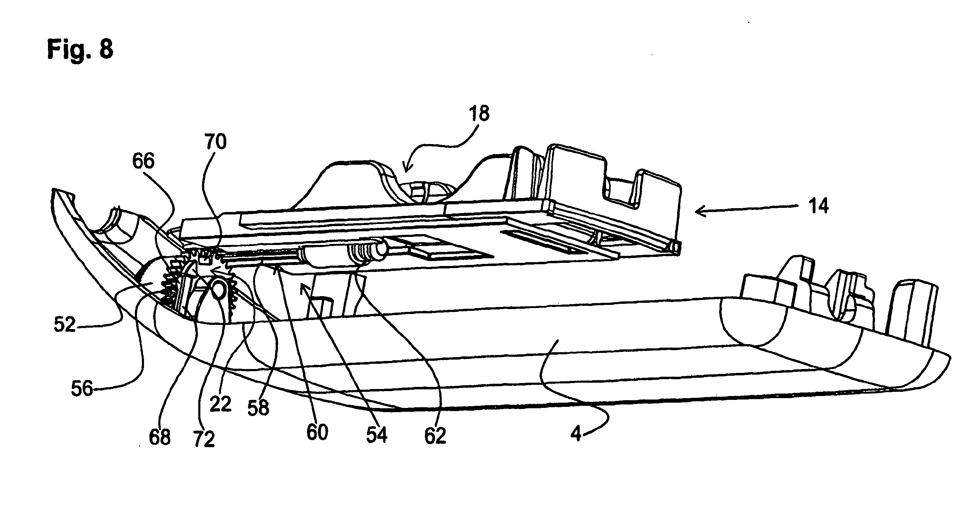

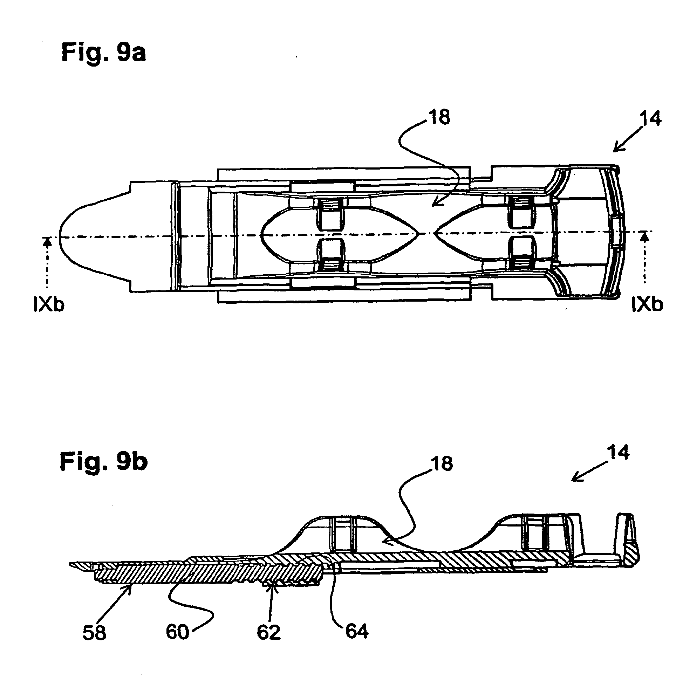

Zwischen dem Stellrad 52 und der Aufnahme 18 ist hierzu ein Stellgetriebe 54 vorgesehen, wie aus Fig. 8 zu entnehmen ist. Dieses weist ein mit dem Stellrad 52 verbundenes Zahnrad 56 auf, das mit einem Zahnradabschnitt 58 einer Spindel 60 kämmt. Diese Spindel 60 ist an einer Unterseite des Einstechschlittens 14 drehbar gelagert. Die Spindel 60 weist ferner einen Gewindeabschnitt 62 auf, der mit einem Mitnahmeelement 64 der Aufnahme 18 in Eingriff steht, wie aus Fig. 9 b zu entnehmen ist.

Durch Verdrehen des Stellrades 52 wird die Spindel 60 somit mittels des Zahnrades 56 verdreht, wodurch wiederum das Mitnahmeelement 64 durch den sich mitdrehenden Gewindeabschnitt 62 der Spindel 60 entlang der Injektionsrichtung R verlagert wird.

Um hierbei einen genauen Wert der Einstechtiefe tE einstellen zu können, sind in das Stellrad 52 mehrere mögliche Werte eingelassen, von denen der jeweils eingestellte Wert von außerhalb des Trägergehäuses 4 ablesbar ist (siehe Fig. 7). Jeder einstellbare Wert entspricht dabei einer bestimmten Position des Stellrades 52 beziehungsweise des Stellgetriebes 54 insgesamt, die durch die Festlegemittel 22 fixierbar ist, wie in Fig. 8 dargestellt.

Hierzu weisen die Feststellmittel 22 bei dieser Ausführungsform der Injektionsvorrichtung 2 eine als Eingriffselement fungierende Rastnase 66 auf, die über einen Federarm 68 am Trägergehäuse 4 gehalten ist (siehe Fig. 8). Diese ist mit jeweils einer von mehreren mit dem Stellrad 52 beziehungsweise mit dem Zahnrad 56 verbundenen Rastaufnahmen 70 verrastbar. Jede auf diese Weise mittels der Rastnase 66 und einer der Rastaufnahmen 70 festlegbaren Positionen des Stellgetriebes 54 entspricht dabei einem der anzeigbaren Werte der Einstechtiefe tE.

Alternativ zu den dargestellten Feststellmitteln 22 können auch die Verzahnungen des Stellgetriebes 54 durch entsprechende Wahl der Zahnwinkel beziehungsweise der Toleranzen so eingestellt werden, dass das Stellgetriebe 54 allein durch Selbsthemmung in einer eingestellten Drehposition verbleibt.

Ferner ist an dem Zahnrad 56 ein Drehanschlag 72 vorgesehen, der in einer Endstellung, wie dargestellt, an dem Federarm 68 anschlägt, wodurch ein Überdrehen des Stellrades 52 vermieden wird.

Nach Einstellen der für die vorgesehene Anwendung gewünschten Einstechtiefe tE kann die Injektionsvorrichtung 2 daraufhin entsprechend der für die erste Ausführungsform beschriebene Vorgehensweise betrieben werden.

## Patentansprüche

1. **Injektionsvorrichtung (2)**
mit einem Trägergehäuse (4), in dem ein Injektionsgerät (8) mit wenigstens einem auspressbaren Injektionsflüssigkeitsbehälter (10) einsetzbar ist,
und einer zur Aktivierung des Injektionsgerätes (8) entlang einer Injektionsrichtung (R) antreibbaren Betätigungsvorrichtung (12), die einen Einstechschlitten (14), eine Aufnahme (18) für das Injektionsgerät (8) sowie einen relativ zum Einstechschlitten (14) verlagerbaren Injektionsschlitten (16) aufweist, der einen Betätigungsstößel (48) für die Beaufschlagung eines Stößels (50) des Injektionsgerätes (8) aufweist,
wobei der Einstechschlitten (14) und der Injektionsschlitten (16) wenigstens zur Durchführung eines Einstechhubes und eines Injektionshubes von einer Kraftbeaufschlagungseinrichtung betätigbar sind und eine Einstechtiefe (tE) des Injektionsgerätes (8) einstellbar ist,
**dadurch gekennzeichnet, dass** die Aufnahme (18) am Einstechschlitten (14) in Injektionsrichtung (R) verstellbar gelagert ist.

2. Injektionsvorrichtung nach Anspruch 1, **dadurch gekennzeichnet, dass** Festlegemittel (22) vorgesehen sind, mittels denen die Aufnahme (18) in einer eingestellten Position gegenüber dem Einstechschlitten (14) festlegbar ist.

3. Injektionsvorrichtung nach Anspruch 2, **dadurch gekennzeichnet, dass** die Festlegemittel (22) wenigstens ein Eingriffselement aufweisen, das in einer von mehreren vorbestimmten Eingriffspositionen mit entsprechenden Eingriffsgegenelementen in Eingriff bringbar ist.

4. Injektionsvorrichtung nach Anspruch 3, **dadurch gekennzeichnet, dass** der Eingriff über einen Verriegelungsschieber (26) herstellbar ist, der zwischen einer Verriegelungsstellung, in der Verriegelungsmittel (28) des Verriegelungsschiebers (26) mit Verriegelungsgegenmitteln (30) des Einstechschlittens (14) in Eingriff stehen, und einer Freigabestellung, in der die Verriegelungsmittel (28) und Verriegelungsgegenmittel (30) außer Eingriff stehen, verschiebbar ist.

5. Injektionsvorrichtung nach Anspruch 4, **dadurch gekennzeichnet, dass** der Verriegelungsschieber (26) in die Verriegelungsstellung vorgespannt ist.

6. Injektionsvorrichtung nach Anspruch 4, **dadurch gekennzeichnet, dass** der Verriegelungsschieber (26) in die Freigabestellung vorgespannt ist.

7. Injektionsvorrichtung nach Anspruch 5 oder 6, **dadurch gekennzeichnet, dass** der Verriegelungsschieber (26) über eine Handhabe (24) verlagerbar ist, die in der Freigabestellung in einen Aufnahmeraum (40) der Aufnahme (18) ragt.

8. Injektionsvorrichtung nach Anspruch 7, **dadurch gekennzeichnet, dass** die Handhabe (24) durch einen ersten Arm (32) eines zweiarmigen Schwenkgliedes (34) gebildet ist, das um eine Schwenkachse (S) herum verschwenkbar gelagert ist und an einem zweiten Arm (36) einen exzentrischen Nocken (38) zur Verlagerung des Verriegelungsschiebers (26) aufweist.

9. Injektionsvorrichtung nach einem der Ansprüche 1 bis 8, **dadurch gekennzeichnet, dass** am Einstechschlitten (14) eine Anzeige (42) zur Bestimmung der aktuell eingestellten Einstechtiefe (tE) vorgesehen ist.

10. Injektionsvorrichtung nach Anspruch 9, **dadurch gekennzeichnet, dass** die Anzeige (42) eine sich in Injektionsrichtung (R) erstreckende Skala (44) aufweist, an der entlang ein Zeigerelement (46) der Aufnahme (18) verschiebbar ist.

11. Injektionsvorrichtung nach einem der Ansprüche 1 bis 3, **dadurch gekennzeichnet, dass** die Aufnahme (18) und der Einstechschlitten (14) über ein Stellgetriebe (54) zueinander verlagerbar sind, das über ein Stellrad (52) betätigbar ist.

12. Injektionsvorrichtung nach Anspruch 11, **dadurch gekennzeichnet, dass** das Stellgetriebe (54) eine Spindel (60) aufweist, die über einen Zahnradabschnitt (58) mit dem Stellrad (52) kämmt und über einen Gewindeabschnitt (62) mit einer Mitnahmekontur (64) der Aufnahme in Eingriff steht und verdrehbar am Einstechschlitten (14) gelagert ist.

13. Injektionsvorrichtung nach Anspruch 12, **dadurch gekennzeichnet, dass** der Zahnradabschnitt (58) in kämmender Position mit dem Stellrad (52) an demselben mittels Gleitbewegung verlagerbar ist.

14. Injektionsvorrichtung nach einem der Ansprüche 11 bis 13, **dadurch gekennzeichnet, dass** das Eingriffselement durch eine gehäuseseitige Rastnase (66) gebildet ist, die mit einer von mehreren in das Stellrad (52) eingelassenen Rastaufnahmen (70) verrastbar ist.

## Claims

1. Injection device (2),
comprising a carrier housing (4) into which an injection instrument (8) with at least one injection-liquid container (10) that can be squeezed out can be inserted,
and an actuation device (12), which can be driven along an injection direction (R) in order to activate the injection instrument (8) and comprises a piercing carriage (14), a receptacle (18) for the injection instrument (8) and an injection carriage (16) which can be moved relative to the piercing carriage (14) and has an actuation plunger (48) for acting on a plunger (50) of the injection instrument (8),
wherein the piercing carriage (14) and the injection carriage (16) can be actuated by a force-application apparatus, at least to carry out a piercing stroke and an injection stroke, and the piercing depth (tE) of the injection instrument (8) can be set,
**characterized in that** the receptacle (18) is adjustably mounted on the piercing carriage (14) in the injection direction (R).

2. Injection device according to Claim 1, **characterized in that** fixing means (22) are provided, by means of which the receptacle (18) can be fixed with respect to the piercing carriage (14) in a set position.

3. Injection device according to Claim 2, **characterized in that** the fixing means (22) have at least one engagement element, which can be made to engage with corresponding engagement counter-elements in one of several predetermined engagement positions.

4. Injection device according to Claim 3, **characterized in that** the engagement can be established by a locking slide (26), which can be displaced between a locked position, in which locking means (28) of the locking slide (26) engage with locking counter-means (30) of the piercing carriage (14), and a release position, in which the locking means (28) and locking counter-means (30) are not in engagement.

5. Injection device according to Claim 4, **characterized in that** the locking slide (26) is pretensioned in the locked position.

6. Injection device according to Claim 4, **characterized in that** the locking slide (26) is pretensioned in the release position.

7. Injection device according to Claim 5 or 6, **characterized in that** the locking slide (26) can be moved by a handle (24), which, in the release position, projects into a receptacle space (40) of the receptacle (18).

8. Injection device according to Claim 7, **characterized in that** the handle (24) is formed by a first arm (32) of a two-armed pivot member (34) which is mounted such that it can pivot about a pivot axis (S) and, on a second arm (36), has an eccentric cam (38) for moving the locking slide (26).

9. Injection device according to one of Claims 1 to 8, **characterized in that** a display (42) for determining the currently set piercing depth (tE) is provided on the piercing carriage (14).

10. Injection device according to Claim 9, **characterized in that** the display (42) has a scale (44) extending in the injection direction (R), along which a pointer element (46) of the receptacle (18) can be displaced.

11. Injection device according to one of Claims 1 to 3, **characterized in that** the receptacle (18) and the piercing carriage (14) can be moved with respect to one another via an adjustment mechanism (54) which is actuatable by an adjustment wheel (52).

12. Injection device according to Claim 11, **characterized in that** the adjustment mechanism (54) has a spindle (60), which meshes with the adjustment wheel (52) via a toothed-wheel section (58) and engages with a pick-up contour (64) of the receptacle via a threaded section (62) and is mounted in a rotatable manner on the piercing carriage (14).

13. Injection device according to Claim 12, **characterized in that** the toothed-wheel section (58) in a meshed position with the adjustment wheel (52) can be moved on the latter by means of a gliding movement.

14. Injection device according to one of Claims 11 to 13, **characterized in that** the engagement element is formed by a housing-side latching lug (66), which can latch into one of several latching receptacles (70) introduced into the adjustment wheel (52).

## Revendications

1. Dispositif d'injection (2)
comprenant un boîtier support (4) dans lequel peut être inséré un appareil d'injection (8) avec au moins un récipient de liquide d'injection (10) pouvant être comprimé,
et un dispositif d'actionnement (12) pouvant être entraîné le long d'une direction d'injection (R) pour activer l'appareil d'injection (8), lequel présente un coulisseau de piquage (14), un logement (18) pour l'appareil d'injection (8) ainsi qu'un coulisseau d'injection (16) pouvant être déplacé par rapport au coulisseau de piquage (14), lequel présente un poussoir d'actionnement (48) pour solliciter un poussoir (50) de l'appareil d'injection (8),
le coulisseau de piquage (14) et le coulisseau d'injection (16) pouvant être actionnés par un dispositif d'application de force au moins pour effectuer une course de piquage et une course d'injection et une profondeur de piquage (tE) de l'appareil d'injection (8) pouvant être ajustée, **caractérisé en ce que** le logement (18) est monté de manière déplaçable dans la direction d'injection (R) sur le coulisseau de piquage (14).

2. Dispositif d'injection selon la revendication 1, **caractérisé en ce que** des moyens de fixation (22) sont prévus, avec lesquels le logement (18) peut être fixé dans une position ajustée par rapport au coulisseau de piquage (14).

3. Dispositif d'injection selon la revendication 2, **caractérisé en ce que** les moyens de fixation (22) présentent au moins un élément d'engagement qui peut être amené en prise dans l'une de plusieurs positions d'engagement prédéterminées avec des éléments d'engagement correspondants.

4. Dispositif d'injection selon la revendication 3, **caractérisé en ce que** l'engagement peut être établi par le biais d'un tiroir de verrouillage (26) qui peut être déplacé entre une position de verrouillage dans laquelle des moyens de verrouillage (28) du tiroir de verrouillage (26) sont en prise avec des moyens de verrouillage conjugués (30) du coulisseau de piquage (14) et une position de libération dans laquelle les moyens de verrouillage (28) et les moyens de verrouillage conjugués (30) ne sont pas en prise les uns avec les autres.

5. Dispositif d'injection selon la revendication 4, **caractérisé en ce que** le tiroir de verrouillage (26) est précontraint dans la position de verrouillage.

6. Dispositif d'injection selon la revendication 4, **caractérisé en ce que** le tiroir de verrouillage (26) est précontraint dans la position de libération.

7. Dispositif d'injection selon la revendication 5 ou 6, **caractérisé en ce que** le tiroir de verrouillage (26) peut être déplacé par le biais d'une manette (24) qui pénètre dans un espace de réception (40) du logement (18) dans la position de libération.

8. Dispositif d'injection selon la revendication 7, **caractérisé en ce que** la manette (24) est formée par un premier bras (32) d'un organe pivotant à deux bras (34) qui est monté de manière à pouvoir pivoter autour d'un axe de pivotement (S) et présente, au niveau d'un deuxième bras (36), une came excentrique (38) pour le déplacement du tiroir de verrouillage (26).

9. Dispositif d'injection selon l'une quelconque des revendications 1 à 8, **caractérisé en ce qu'**un affichage (42) pour déterminer la profondeur de piquage actuellement ajustée (tE) est prévu sur le coulisseau de piquage (14).

10. Dispositif d'injection selon la revendication 9, **caractérisé en ce que** l'affichage (42) présente une échelle (44) s'étendant dans la direction d'injection (R) le long de laquelle un élément d'aiguille (46) du logement (18) peut être déplacé.

11. Dispositif d'injection selon l'une quelconque des revendications 1 à 3, **caractérisé en ce que** le logement (18) et le coulisseau de piquage (14) peuvent être déplacés l'un par rapport à l'autre par le biais d'un mécanisme de commande (54) qui peut être actionné par le biais d'une molette de commande (52).

12. Dispositif d'injection selon la revendication 11, **caractérisé en ce que** le mécanisme de commande (54) présente une broche (60) qui s'engrène par le biais d'une section de roue dentée (58) avec la molette de commande (52) et qui est en prise par le biais d'une section filetée (62) avec un contour d'entraînement (64) du logement et qui est montée de manière rotative sur le coulisseau de piquage (14).

13. Dispositif d'injection selon la revendication 12, **caractérisé en ce que** la section de roue dentée (58) peut être déplacée dans la position d'engrènement avec la molette de commande (52) sur celle-ci par le biais d'un mouvement de glissement.

14. Dispositif d'injection selon l'une quelconque des revendications 11 à 13, **caractérisé en ce que** l'élément d'engagement est formé par un ergot d'encliquetage (66) du côté du boîtier, lequel peut être encliqueté avec l'un de plusieurs logements d'encliquetage (70) pratiqués dans la molette de commande (52).
